# EUROPÄISCHE PATENTSCHRIFT

(11) **EP 1 738 743 B1**
(45) Veröffentlichungstag und Bekanntmachung des Hinweises auf die Patenterteilung: **14.11.2012**
(21) Anmeldenummer: 06116027.1
(22) Anmeldetag: 26.06.2006
(51) Int. Cl.: A01N 47/16, A01N 25/12, A61K 8/49, A61Q 17/02, A61K 8/25, A61K 8/26, A61K 8/73, A61K 8/81, A61K 8/86, A61K 8/87

(54) **Insektenschutzmittel**
Insect repellent
Produit insectifuge

(30) Priorität: 27.06.2005 DE 102005030015
(43) Veröffentlichungstag der Anmeldung: 03.01.2007
(73) Patentinhaber: Beiersdorf AG, 20253 Hamburg (DE)
(72) Erfinder: Schulz, Jens, 22869 Schenefeld (DE); Von der Fecht, Stephanie, 22869 Schenefeld (DE); Nielsen, Jens, 25448 Henstedt-Ulzburg (DE); Kröpke, Rainer, 22869 Schenefeld (DE)

(56) Entgegenhaltungen:
- WO-A-03/020232
- DE-A1- 10 349 666

## Beschreibung

Die vorliegende Erfindung betrifft eine kosmetische Zubereitung enthaltend einen oder mehrere Repellent-Wirkstoffe mit einem Dampfdruck von mindestens 3,2 · 10⁻⁴ hPa bei 20 °C und partikuläre Füllstoffe mit einer spezifischen Oberfläche von mehr als mehr als 0,2 m²/g.

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenschutzmittel" nicht nur solche Präparate zu verstehen, die gegen Insekten (insbesondere Mücken) wirksam sind. Vielmehr gilt das nachstehend Gesagte selbstverständlich auch für solche Präparate, die andere Blut saugende oder beißende Parasiten und/oder Lästlinge (z.B. Spinnen, Flöhe, Milben) abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hoch siedende Flüssigkeiten oder niedrig schmelzende bzw. sublimierende kristalline Stoffe, die bei Raumtemperatur langsam verdampfen. Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur 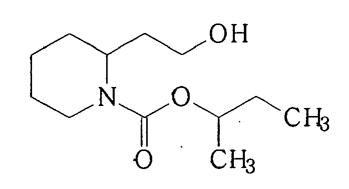 und einen bei 20 °C einen Dampfdruck von 3,4 10⁻⁴ hPa aufweist. 1-Piperidincarboxylsäure 2-(2-hydroxyethyl)-1-methylpropylester gilt als besonders wirksamer Repellent-Wirkstoff. Insektenabwehrmittel des Standes der Technik, die mit 1-Piperidincarboxylsäure 2-(2-hydroxyethyl)-1-methylpropylester formuliert werden, weisen jedoch eine Reihe von Nachteilen auf:
- Aufgrund des Dampfdruckes von 1-Piperidincarboxylsäure 2-(2-hydroxyethyl)-1-methylpropylester, sind die Zubereitungen nur begrenzte Zeit (2 bis 6 Stunden) gegen Insekten wirksam.
- Zubereitungen mit 1-Piperidincarboxylsäure 2-(2-hydroxyethyl)-1-methylpropylester wirken auf der Haut klebrig.
- Da der Wirkstoff im Normalzustand (20 °C, 1 bar) flüssig und relativ unpolar ist, besteht bei dünnwandigen Packmitteln aus Polyethylen (PE), Polypropylen (PP) oder Polyethylenterephthalat (PET) die Gefahr, das der Wirkstoff über einen längeren Zeitraum durch den Kunststoff hindurch entweicht. Dies kann zu einem Wirkungsverlust der Zubereitung und einem Ablösen der Packungsbedruckung führen. Um diesen Effekt zu unterdrücken, müssen relativ dickwandige Verpackungsbehältnisse (hoher Kunststoffverbrauch!) eingesetzt werden.

Es war daher die Aufgabe der vorliegenden Erfindung, die Mängel des Standes der Technik zu beseitigen und ein Insektenabwehrmittel zu entwickeln, dass nach dem Auftragen auf die Haut und/oder den Haaren besonders lang anhaltend wirksam ist, eine besonders angenehme, nicht klebrige Sensorik aufweist und sich problemlos in Verpackungsbehältnissen aus Kunststoff aufbewahren lässt.

Überraschend gelöst werden die Aufgaben durch
Eine kosmetische Zubereitung enthaltend
a) Icaridin
b) partikuläre Füllstoffe mit einer spezifischen Oberfläche von mehr als mehr als 0,2 m²/g, gewählt aus der Gruppe Nylon, Talkum, Polyethylene und Cellulose.

Ferner werden die Aufgaben überraschend gelöst durch ein Kosmetikum aus
a) einem Packmittel aus Polyethylen (PE), Polypropylen (PP) oder Polyethylenterephthalat (PET) und
b) einer kosmetischen Zubereitung enthaltend Icaridin
partikuläre Füllstoffe mit einer spezifischen Oberfläche von mehr als mehr als 0,2 m²/g wie oben definiert.

Erfindungsgemäß ist ferner die Verwendung von den bestimmten Füllstoffen mit einer spezifischen Oberfläche von mindestens 0,2 m²/g zur Verlängerung der Insekten abweisenden Wirksamkeitsdauer kosmetischer Zubereitungen mit einem Gehalt an dem Repellent-Wirkstoff Icaridin bei deren Anwendung auf der menschlichen Haut, den menschlichen Haaren und/oder bei ihrer Lagerung in Packmitteln aus Polyethylen, Polypropylen oder Polyethylenterephthalat.

Mit Hilfe der erfindungsgemäßen Zubereitungen können jetzt Packmittel mit im Vergleich zum Stand der Technik dünneren Packungswänden eingesetzt werden, wodurch sich der Materialaufwand für die Verpackung reduziert.

Erfindungsgemäß ist nicht zuletzt die Verwendung von den bestimmten Füllstoffen mit einer spezifischen Oberfläche von mindestens 0,2 m²/g zur Reduzierung der Klebrigkeit von kosmetischen Zubereitungen mit einem Gehalt an dem Repellent-Wirkstoff Icaridin.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, dass die Konzentration an dem Repellent-Wirkstoff Icaridin in der kosmetischen Zubereitung von 1 bis 40 Gewichts-% und bevorzugt von 10 bis 20 Gewichts-%, jeweils bezogen auf das Gesamtgewicht der kosmetischen Zubereitung, beträgt.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, dass die Konzentration an Füllstoffen mit einer spezifischen Oberfläche von mehr als mehr als 0,2 m²/g in der kosmetischen Zubereitung von 0,5 bis 20 Gewichts-% und bevorzugt von 1 bis 10 Gewichts-%, jeweils bezogen auf das Gesamtgewicht der kosmetischen Zubereitung, beträgt.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, dass das Gewichtsverhältnis von Repellent-Wirkstoff Icaridin in der kosmetischen Zubereitung von 10 :1 bis 1:1 und bevorzugt von 10:1 bis 5:2 beträgt.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, dass der Füllstoff mit einer spezifischen Oberfläche von mehr als mehr als 0,2 m²/g eine durchschnittliche Partikelgröße von 0,01-100 µm aufweist.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, dass der Füllstoff mit einer spezifischen Oberfläche von mehr als mehr als 0,2 m²/g eine Dichte von 0,02-3,16 g/cm³ aufweist.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, dass als Füllstoff mit einer spezifischen Oberfläche von mehr als mehr als 0,2 m²/g eine oder mehrere Stoffe gewählt aus der Gruppe Nylon, sowie Polyethylen.

Die erfindungsgemäße kosmetische Zubereitung, das erfindungsgemäße Kosmetikum und die erfindungsgemäßen Verwendungen sind erfindungsgemäß vorteilhaft dadurch gekennzeichnet, das die Zubereitung in Form einer wässrigen oder wässrig-alkoholischen Lösung, einer Emulsion (W/O, O/W, W/S, S/W oder multiple Emulsion), einer Dispersion, einer Pickering-Emulsion vorliegt. Dabei ist es erfindungsgemäß bevorzugt, wenn die kosmetische Zubereitung in Form einer Emulsion vorliegt.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben den erfindungsgemäßen Repellent-Wirkstoffen kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alphaglycosylrutin und/oder in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Das erfindungsgemäße Packmittel liegt erfindungsgemäß vorteilhaft in Form einer Tube oder einer Kunststoff-Flasche vor. Dabei sind Kunststoff-Flaschen erfindungsgemäß bevorzugt. Eine erfindungsgemäß besonders bevorzugte Ausführungsform stellen Kunststoff-Flaschen mit aufgesetztem Pumpspender dar, mit welchem die erfindungsgemäße Zubereitung aus dem Packmittel gefördert und ggf. versprüht werden kann.

Es ist erfindungsgemäß bevorzugt, wenn das erfindungsgemäße Kosmetikum aus einem Packmittel aus Polyethylen (PE) gebildet wird. Erfindungsgemäß besonders bevorzugt ist dabei der Einsatz von "high density polyethylene"(HDPE nach DIN 7728, TI. 1, 01/1988).

Erfindungsgemäß ist insbesondere die Verwendung der erfindungsgemäßen Zubereitung zur Prophylaxe von Mückenstichen und Zeckenbissen.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

### W/O-Emulsionen

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 7,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Cellulose | 0,5 | 1,0 | --- | 0,25 | 2,5 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| PTFE | --- | --- | 1,5 | --- | --- |
| Icaridin | 5 | 7,5 | 10 | 12,5 | 30 |
| Wasser | ad 100 | ad 100 | ad 100 | ad 100 | ad 100 |

### W/O-Emulsionen

| | **6** | **7** | **8** | **9** | **10** |
|---|---|---|---|---|---|
| PEG-30 Dipolyhydroxystearat | --- | 0,5 | 0,25 | --- | 8,0 |
| Lanolin Alcohol | 1,0 | 1,5 | 1,75 | 3,0 | -- |
| Paraffinöl | 12,5 | 10,0 | 8,0 | 5,0 | 12,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Kaliumsorbat | 0,3 | 0,4 | 0,25 | 0,15 | --- |
| Polyurethan | 1,5 | --- | 1,0 | 2,5 | 10 |
| Icaridin | 7,5 | 12,5 | 17,5 | 20 | 35 |
| Talkum | 1,5 | 3,5 | --- | --- | --- |
| Wasser | ad 100 | ad 100 | ad 100 | ad 100 | ad 100 |

### W/S-Emulsion

| | **11** | **12** | **13** | **14** | **15** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Cetyldimethicon | 0,5 | --- | 0,7 | --- | --- |
| Benzylalkohol | --- | --- | 0,05 | --- | 0,1 |
| Polyethylen | 0,3 | 6 | 1,25 | 2,0 | 7,5 |
| Icaridin | 3 | 15 | 12,5 | 20 | 7,5 |
| Wasser | ad 100 | ad 100 | ad 100 | ad 100 | ad 100 |

### W/S-Emulsionen

| | **16** | **17** | **18** | **19** | **20** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Icaridin | 3,0 | 10 | 12,5 | 15 | 30 |
| Stearyldimethicon | 0,5 | --- | 0,7 | --- | --- |
| Dehydracetsäure | --- | --- | 0,05 | --- | 0,1 |
| Tapiocastärke | 0,3 | 2,0 | 12,5 | 1,5 | 3 |
| Wasser | ad 100 | ad 100 | ad 100 | ad 100 | ad 100 |

### W/O-Emulsionen

| | **21** | **22** | **23** | **24** | **25** |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Talkum | 0,5 | 4 | 1,5 | 2,0 | 10 |
| Icaridin | 5 | 10 | 15 | 20 | 25 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | --- | --- | 0,05 | --- | 0,1 |
| Wasser | ad 100 | ad 100 | ad 100 | ad 100 | ad 100 |

### W/O-Emulsionen

| | **26** | **27** | **28** | **29** | **30** |
|---|---|---|---|---|---|
| Polyglyceryl-2-dipolyhydroxystearat | 3,0 | --- | 0,25 | --- | 6,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Cellulose | 1,618 | 1,618 | 0,75 | 1,5 | 4 |
| Icaridin | 1,618 | 16,18 | 7,5 | 15 | 40 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

### Silikon in Wasser-Emulsion

| | **31** | **32** | **33** | **34** | **35** |
|---|---|---|---|---|---|
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Talkum | 1 | 4 | 8 | 4 | 1,5 |
| Icaridin | 10 | 20 | 30 | 40 | 15 |
| Xanthan Gum | --- | 0,1 | -- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Wasser | ad 100 | ad 100 | ad 100 | ad 100 | ad 100 |

### O/W-Emulsion

| | **36** | **37** | **38** | **39** | **40** |
|---|---|---|---|---|---|
| Glycerylsterat | 1,0 | --- | -- | 0,5 | 0,75 |
| Polyethylenglycol(40)stearat | 10,0 | --- | 5 | -- | -- |
| Triglycerinmethylglucosedistearat | --- | 5,5 | --- | --- | 3,5 |
| Sorbitanstearat | --- | 1,5 | 3 | --- | --- |
| Cyclomethicon | 2,5 | 15 | 8,0 | 5,0 | 7,5 |
| Dimethicon | 5,0 | 3,0 | 5,0 | 2,0 | 5,0 |
| Behenylalkohol | 1 | --- | 2 | 1 | --- |
| Stearylalkohol | --- | 1 | --- | 1 | --- |
| Ginkgo Biloba | 0,1 | 0,5 | 1,0 | 2,5 | 1,0 |
| Icaridin | 7,5 | 15 | 12,5 | 20 | 25 |
| Cetylstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Glycerin | 5 | 10 | 3 | 15 | 7,5 |
| Nylon | 0,75 | 1,5 | 5 | 2,0 | 10 |
| Wasser | ad 100 | ad 100 | ad 100 | ad 100 | ad 100 |

### O/W-Emulsion

| | **41** | **42** | **43** | **44** | **45** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Behenylalkohol | 3 | 2 | | --- 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Icaridin | 10 | 20 | 15 | 7,5 | 5,0 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Silikat | 1 | 2 | 1,5 | 3 | 0,5 |
| Wasser | ad 100 | ad 100 | ad 100 | ad 100 | ad 100 |

### O/W-Emulsion

| | **46** | **47** | **48** | **49** | **50** |
|---|---|---|---|---|---|
| Glycerylsteratcitrat | 1,0 | 0,5 | 1,5 | 9,0 | 0,3 |
| Polyethylenglycol(20)cetearylether | 10,0 | 1,0 | 5 | -- | -- |
| Triglycerinmethylglucosedistearat | --- | --- | 3,5 | --- | 2,5 |
| PMMA | 0,75 | 1,5 | 2,25 | 12 | 3,75 |
| Icaridin | 7,5 | 15 | 22,5 | 30 | 37,5 |
| Dimethicon | 0,5 | 3,0 | 0,75 | 1,5 | 0,2 |
| Behenylalkohol | 1 | --- | 2 | --- | 0,2 |
| Dicaprylylcarbonat | 3 | 5 | 10 | 5 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| lodopropynylbutylcarbamat | --- | --- | 0,05 | --- | 0,1 |
| Phenoxyethanol | --- | 0,5 | --- | 0,15 | --- |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

### O/W-Emulsion

| | **51** | **52** | **53** | **54** | **55** |
|---|---|---|---|---|---|
| Glycerylsterat | 2,6 | --- | --- | 2,7 | 2,6 |
| Caprylic/Capric Triglycerid | 3,5 | 4,0 | 3,75 | 3,1 | 5,5 |
| Dicaprylylcarbonat | 3,5 | 2,5 | 3,75 | --- | 3,0 |
| Triglycerinmethylglucosedistearat | --- | 3,0 | 3,0 | --- | --- |
| Cetystearylalkohol | 3,0 | --- | --- | 4,0 | 3,5 |
| Dimethicon | --- | 3,0 | 5,0 | 3,0 | 2,75 |
| Cetylalkohol | --- | 3,0 | 1,5 | 1,25 | --- |
| Polyethylenglycol(40)stearat | 0,8 | --- | --- | 1,3 | 1,3 |
| C12-15 Alkylbenzoat | --- | 2,5 | 3,75 | 3,0 | 4,5 |
| PTFE | 0,5 | 1,0 | 1,5 | 4 | 2,5 |
| Icaridin | 5 | 10 | 15 | 20 | 25 |
| Natriumoleanolat | 0,1 | 0,1 | 0,1 | 0,075 | 0,1 |
| Cyclomethicon | 3,5 | --- | --- | --- | 0,25 |
| Propylenglykol | 0,5 | 0,5 | 0,5 | 0,35 | 0,2 |
| Mineralöl | --- | --- | --- | 6,0 | --- |
| Phenoxyethanol | 0,4 | 0,4 | 0,4 | 0,75 | 1,25 |
| Zitronensäure | --- | --- | 0,05 | --- | --- |
| Ethanol | --- | --- | --- | 3,0 | --- |
| Ethylparaben | --- | --- | --- | 0,3 | --- |
| Methylparaben | 0,2 | 0,2 | 0,2 | 0,2 | 0,2 |
| Glycerin | 10,0 | 10,0 | 10,0 | 10,0 | 12,5 |
| Propylparaben | 0,1 | 0,1 | 0,1 | 0,1 | 0,1 |
| Carbomer, Natrium-Salz | 0,1 | 0,1 | 0,1 | 0,2 | 0,1 |
| Parfum | q,s, | q,s, | q,s, | q,s, | q,s, |
| lodopropynylbutylcarbamat | 0,018 | 0,018 | 0,018 | --- | 0,018 |
| Wasser | ad 100 | ad 100 | ad 100 | ad 100 | ad 100 |

### Pumpspray

| | **56** | **57** | **58** | **59** | **60** |
|---|---|---|---|---|---|
| Polyethylenglycol(40)stearat | 1,0 | --- | 0,5 | -- | -- |
| Cyclomethicon | 0,5 | --- | --- | --- | --- |
| Icaridin | 2 | 10 | 15 | 20 | 40 |
| Parfum | q,s, | q,s, | q,s, | q,s, | q,s, |
| Ethanol | 15 | 20 | 30 | 40 | 20 |
| Methylparaben | 0,4 | 0,1 | 0,05 | 0,3 | 0,4 |
| Meeresextrakt | --- | --- | 0,1 | --- | --- |
| Aloe Vera Extrakt | 0,1 | --- | 0,1 | --- | --- |
| Hammamelis-Extrakt | 0,1 | --- | 0,1 | 5 | 0,5 |
| Glycerin | 5 | 10 | 3 | 15 | 7,5 |
| Talkum | 1,0 | 2,5 | 3,5 | 0,15 | 7,5 |
| Wasser | ad 100 | ad 100 | ad 100 | ad 100 | ad 100 |

### Spray mit Repellent

| | **61** | **62** | **63** | **64** | **65** |
|---|---|---|---|---|---|
| Ethylbutylacetylaminopropionat | 5 | 35 | 15 | 20 | 7,5 |
| N, N-Diethyltoluolamid | 5 | --- | -- | 1 | -- |
| Icaridin | 5 | 3,0 | 2,0 | 1,5 | 12,5 |
| 1-(3-Cyclohexen-1-yl-carbonyl)-2-methylpiperidin | 5 | --- | 2 | 1 | --- |
| Hammamelis-Extrakt | 0,2 | --- | --- | 1 | 0,2 |
| Aloe Vera Extrakt | --- | --- | 1 | 1 | 5,0 |
| Tocopherol | 0,5 | 0,5 | 0,75 | 0,25 | 0,1 |
| Meeresextrakt | 0,5 | --- | 0,75 | 3,0 | 0,25 |
| Panthenol | 0,5 | --- | 0,75 | 0,25 | 0,1 |
| Nylon | 1 | 1,5 | 0,5 | 0,75 | 10 |
| Parfum | q,s, | q,s, | q,s, | q,s, | q,s, |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

## Patentansprüche

1. Kosmetische Zubereitung enthaltend
a) 1-Piperidincarboxylsäure 2-(2-hydroxyethyl)-1-methylpropylester (INN: Icaridin) und
b) partikuläre Füllstoffe mit einer spezifischen Oberfläche von mehr als mehr als 0,2 m²/g gewählt aus der Gruppe Nylon, Talkum, Polyethylene und Cellulose.

2. Kosmetikum aus
a) einem Packmittel aus Polyethylen, Polypropylen oder Polyethylenterephthalat und
b) einer kosmetischen Zubereitung enthaltend 1-Piperidincarboxylsäure 2-(2-hydroxyethyl)-1-methylpropylester (INN: Icaridin) und partikuläre Füllstoffe mit einer spezifischen Oberfläche von mehr als mehr als 0,2 m²/g gewählt aus der Gruppe Nylon, Talkum, Polyethylene und Cellulose.

3. Verwendung von Füllstoffen mit einer spezifischen Oberfläche von mindestens 0,2 m²/g gewählt aus der Gruppe Nylon, Talkum, Polyethylene und Cellulose, zur Verlängerung der Insekten abweisenden Wirksamkeitsdauer kosmetischer Zubereitungen mit einem Gehalt an 1-Piperidincarboxylsäure 2-(2-hydroxyethyl)-1-methylpropylester (INN: Icaridin), bei deren Anwendung auf der menschlichen Haut, den menschlichen Haaren und/oder bei ihrer Lagerung in Packmitteln aus Polyethylen, Polypropylen oder Polyethylenterephthalat.

4. Verwendung von Füllstoffen mit einer spezifischen Oberfläche von mindestens 0.2 m²/g gewählt aus der Gruppe Nylon, Talkum, Polyethylene und Cellulose, zur Reduzierung der Klebrigkeit von kosmetischen Zubereitungen mit einem Gehalt an 1-Piperidincarboxylsäure 2-(2-hydroxyethyl)-1-methylpropylester (INN: Icaridin).

5. Kosmetische Zubereitung, Kosmetikum oder Verwendung nach einem der vorhergehenden Ansprüche, **dadurch gekennzeichnet, dass** die Konzentration an 1-Piperidincarboxylsäure 2-(2-hydroxyethyl)-1-methylpropylester (INN: Icaridin) in der kosmetischen Zubereitung von 1 bis 40 Gewichts-%, bezogen auf das Gesamtgewicht der kosmetischen Zubereitung, beträgt.

6. Kosmetische Zubereitung. Kosmetikum oder Verwendung nach einem der vorhergehenden Ansprüche, **dadurch gekennzeichnet, dass** die Konzentration an Füllstoffen mit einer spezifischen Oberfläche von mehr als mehr als 0,2 m²/g in der kosmetischen Zubereitung von 1 bis 30 Gewichts-%, bezogen auf das Gesamtgewicht der kosmetischen Zubereitung, beträgt.

7. Pflaster oder Tuch getränkt mit einer kosmetischen Zubereitung nach einem der vorhergehenden Ansprüche.

## Claims

1. Cosmetic preparation comprising
a) 1-piperidinecarboxylic acid 2-(2-hydroxyethyl)-1-methylpropyl ester (INN: Icaridine) and
b) particulate fillers with a specific surface area of more than 0.2 m²/g selected from the group nylon, talc, polyethylene and cellulose.

2. Cosmetic comprising
a) a package made of polyethylene, polypropylene or polyethylene terephthalate and
b) a cosmetic preparation comprising 1-piperidinecarboxylic acid 2-(2-hydroxyethyl)-1-methylpropyl ester (INN: Icaridine) and particulate fillers with a specific surface area of more than 0.2 m²/g selected from the group nylon, talc, polyethylene and cellulose.

3. Use of fillers with a specific surface area of at least 0.2 m²/g selected from the group nylon, talc, polyethylene and cellulose for prolonging the insect-repelling effectiveness time of cosmetic preparations containing 1-piperidinecarboxylic acid 2-(2-hydroxyethyl)-1-methylpropyl ester (INN: Icaridine) upon their use on the human skin, the human hair and/or during their storage in packagings made of polyethylene, polypropylene or polyethylene terephthalate.

4. Use of fillers with a specific surface area of at least 0.2 m²/g selected from the group nylon, talc, polyethylene and cellulose for reducing the stickiness of the cosmetic preparations containing 1-piperidinecarboxylic acid 2-(2-hydroxyethyl)-1-methylpropyl ester (INN: Icaridine).

5. Cosmetic preparation, cosmetic or use according to one of the preceding claims, **characterized in that** the concentration of 1-piperidinecarboxylic acid 2-(2-hydroxyethyl)-1-methylpropyl ester (INN: Icaridine) in the cosmetic preparation is from 1 to 40% by weight, based on the total weight of the cosmetic preparation.

6. Cosmetic preparation, cosmetic or use according to one of the preceding claims, **characterized in that** the concentration of fillers with a specific surface area of more than 0.2 m²/g in the cosmetic preparation is from 1 to 30% by weight, based on the total weight of the cosmetic preparation.

7. Plaster or wipe impregnated with a cosmetic preparation according to one of the preceding claims.

## Revendications

1. Préparation cosmétique contenant
a) du 1-pipéridinecarboxylate de 2-(2-hydroxyéthyl)-1-méthylpropyle (INN : icaridine) et
b) des charges particulaires ayant une surface spécifique de plus de 0,2 m²/g, choisies dans le groupe constitué par le Nylon, le talc, les polyéthylènes et la cellulose.

2. Cosmétique constitué
a) d'un emballage à base de polyéthylène, polypropylène ou poly(éthylénetéréphtalate) et
b) d'une préparation cosmétique contenant du 1-pipéridinecarboxylate de 2-(2-hydroxyéthyl)-1-méthylpropyle (INN : icaridine) et
des charges particulaires ayant une surface spécifique de plus de 0,2 m²/g, choisies dans le groupe constitué par le Nylon, le talc, les polyéthylènes et la cellulose.

3. Utilisation de charges ayant une surface spécifique d'au moins 0,2 m²/g, choisies dans le groupe constitué par le Nylon, le talc, les polyéthylènes et la cellulose, pour le prolongement de la durée de l'activité insectifuge de préparations cosmétiques ayant une teneur en 1-pipéridinecarboxylate de 2-(2-hydroxyéthyl)-1-méthylpropyle (INN : icaridine), dans leur application sur la peau humaine, les cheveux humains et/ou dans leur stockage dans des emballages à base de polyéthylène, polypropylène ou poly(éthylène-téréphtalate).

4. Utilisation de charges ayant une surface spécifique d'au moins 0,2 m²/g, choisies dans le groupe constitué par le Nylon, le talc, les polyéthylènes et la cellulose, pour la réduction de la pégosité de préparations cosmétiques ayant une teneur en 1-pipéridinecarboxylate de 2-(2-hydroxyéthyl)-1-méthylpropyle (INN : icaridine).

5. Préparation cosmétique, cosmétique ou utilisation selon l'une quelconque des revendications précédentes, **caractérisés en ce que** la concentration du 1-pipéridinecarboxylate de 2-(2-hydroxyéthyl)-1-méthylprvpyle (INN : icaridine) dans la préparation cosmétique vaut de 1 à 40 % en poids, par rapport au poids total de la préparation cosmétique.

6. Préparation cosmétique, cosmétique ou utilisation selon l'une quelconque des revendications précédentes, **caractérisés en ce que** la concentration des charges ayant une surface spécifique de plus de 0,2 m²/g dans la préparation cosmétique vaut de 1 à 30 % en poids, par rapport au poids total de la préparation cosmétique.

7. Pansement ou lingette imprégnés avec une préparation cosmétique selon l'une quelconque des revendications précédentes.
